(19) Europäisches Patentamt
European Patent Office
Office européen des brevets

(11) Veröffentlichungsnummer: **0 024 682**
**B1**

# (12) EUROPÄISCHE PATENTSCHRIFT

(45) Veröffentlichungstag der Patentschrift: **22.12.82**

(51) Int. Cl.³: **C 07 C 103/133,** C 07 C 103/127, C 07 C 102/02

(21) Anmeldenummer: **80104943.8**

(22) Anmeldetag: **20.08.80**

## (54) Verfahren zur Herstellung von n-Propyl-n-propylidenacetamid oder Di-n-propylacetamid.

(30) Priorität: **21.08.79 DE 2933759**

(43) Veröffentlichungstag der Anmeldung: **11.03.81 Patentblatt 81/10**

(45) Bekanntmachung des Hinweises auf die Patenterteilung: **22.12.82 Patentblatt 82/51**

(84) Benannte Vertragsstaaten: **BE CH DE FR GB IT LI NL**

(56) Entgegenhaltungen:
**DE-C-749 643**
**Methoden der organischen Chemie (Heuben-Weyl), Band VIII, Seiten 274 bis 277; Catalytic Hydrogenation in Organic Synthesis Procedures and Commentary (Morris Freifelder), Seite 16.**
**Die Akte enthält technische Angaben, die nach dem Eingang der Anmeldung eingereicht wurden und die nicht in dieser Patenschrift enthalten sind.**

(73) Patentinhaber: **Consortium für elektrochemische Industrie GmbH, Zielstattstrasse 20, D-8000 München 70 (DE)**

(72) Erfinder: **Gebauer, Helmut, Dr. Dipl.-Chem., *Schaffhauser Strasse* 18/7, D-8000 München 71 (DE)**

EP 0 024 682 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von n-Propyl-n-propylidenacetamid oder Di-n-propylacetamid

Die vorliegende Erfindung bezieht sich auf ein neues Verfahren zur Herstellung von n-Propyl-n-propylidenacetamid der Formel

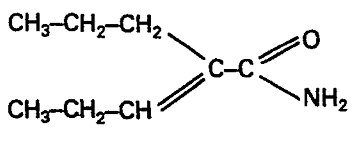

oder Di-n-propylacetamid der Formel

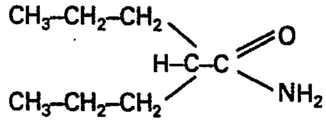

Di-n-propylacetamid ist ein bekanntes Produkt mit pharmakologischen Eigenschaften (DOS 27 21 261, Seite 2, 3. Absatz) und insbesondere aussergewöhnlichen antikonvulsiven Eigenschaften. Zur Zeit wird Di-n-propylacetamid in weitem Masse als antiepileptisches Mittel verwendet. Hinsichtlich dieses Mittels erweist sich n-Propyl-n-propylidenacetamid als wertvolles Zwischenprodukt zur Herstellung des obengenannten Pharmazeutikums. Daneben fungiert es selbst als physiologisch ebenso wirksame Substanz wie Di-n-propylacetamid (E. Taillandier et al., Arch. Pharm. 310, 1977, Seite 400, Zeilen 1 bis 7).

Die bisher gängigen Verfahren zur Herstellung von Di-n-propylacetamid beinhalten zumeist die umständliche, mit vielen Reaktionsstufen verbundene Synthese über Malonester oder Cyanessigester.

So wird in der DOS 27 21 261 angegeben, dass eines der zweckmässigsten und am häufigsten angewendeten Verfahren von Malonsäurediäthylester ausgeht (DOS 27 21 261, Seite 3, Absatz 2). Dieser ist wiederum eine kostspielige Substanz, bekannt aufwendig in der Herstellung, der zudem den nicht zu übersehenden Nachteil mit sich bringt, dass auf umständliche Art und Weise mittels 7 Reaktionsstufen (DOS 27 21 261, Seite 3, Absatz 4, 4. Zeile) das Endprodukt synthetisiert wird.

Ein weiterer Weg zur Herstellung von Di-n-propylacetamid geht von Cynaessigester aus (DOS 27 21 161, Seite 6, Zeile 3), der auch lediglich durch eine aufwendige Verfahrensweise zu erhalten ist und zudem kostspielig ist, was gleichermassen für die Alkylbromide oder Jodide gilt, die zur Alkylierung der genannten Ausgangssubstanzen erforderlich sind. Die Herstellung von Di-n-propylacetamid gemäss letzteren Verfahrens erfordert 5 Stufen mit zumeist längeren Reaktionszeiten und hohen Temperaturen (DOS 27 21 161, Seite 13, 4. Absatz, Zeilen 1 bis 5).

Weiterhin sind aufwendige Isolierungs- und Reinigungsschritte zu unternehmen. So kann die Eliminierung von 2-Allylvalerolacton und Diallylmalonsäure (DOS 27 61 261, Seite 4, Zeilen 1 bis 3) erforderlich werden. Es fallen bei diesen Verfahren monoalkylierte Produkte an (0,3–5%), die zu der übelriechenden Valeriansäure – entstanden nach der Decarboxylierung und Verseifung im Verfahren – führen können, mit denen das Endprodukt behaftet ist.

Bei den gängigen Verfahren geht man von einer $C_3$-Einheit aus und knüpft zwei weitere $C_3$-Einheiten an. Das überzählige neunte C-Atom muss über die Decarboxylierung wieder entfernt werden und geht damit verloren.

Es wurde nun ein Verfahren zur Herstellung von n-Propyl-n-propylidenacetamid oder Di-n-Propylacetamid durch säurekatalysierte Umlagerung von aus Keton hergestelltem Cyanhydrin und gegebenenfalls Hydrierung des ungesättigten Carbonsäureamids gefunden, das dadurch gekennzeichnet ist, dass

a) Di-n-propylketon in der ersten Stufe mit einem Alkalicyanid oder Blausäure umgesetzt und

b) das entstehende Di-n-propylketoncyanhydrin in der Weise zu n-Propyl-n-propylidenacetamid umgelagert wird, dass das Cyanhydrin konzentrierter Mineralsäure bei 20–60 °C zugesetzt, anschliessend daran auf eine Temperatur von 75–90 °C erhitzt und schliesslich das entstandene Reaktionsprodukt noch auf 125–140 °C belassen wird, sowie gegebenenfalls nach Abkühlung und Isolierung des Produktes.

c) letzteres zum Di-n-propylacetamid hydriert wird.

Mit den beanspruchten Verfahren werden die beschriebenen Nachteile beseitigt.

Das Ausgangsmaterial Di-n-propylketon ist leicht mit sehr guter Ausbeute durch Ketonisierung der grosstechnischen Produkte Butanol, Butyraldehyd oder Buttersäure zugänglich, und es gelingt mit einfachen Operationen einen begehrten Wirkstoff herzustellen. Beschränkt man sich auf das nach neueren Untersuchungen von Taillandier physiologisch ebenso wirksame ungesättigte Acetamid, sind sogar nur zwei Stufen erforderlich.

Das erfindungsgemässe Verfahren geht von einem Ausgangsprodukt aus, das grosstechnisch verfügbar und kostengünstig ist. Das Verfahren umfasst drei Stufen und ist somit gegenüber dem Stand der Technik der fünf bis sieben Reaktionsstufen benötigt, wirtschaftlicher. Die Reaktionszeiten insgesamt sowie die der einzelnen Stufen sind kürzer.

Das vorliegende Verfahren arbeitet mit vorteilhaften , leichter handhabbaren Temperaturen, liefert keine störenden Mengen an Nebenprodukten, gibt zufriedenstellende Ausbeuten und besondere Produktreinheit ohne Anwendung umständlicher Massnahmen.

Bisher wurde weitgehend vermieden, die prinzipiell bekannte (vgl. z.B. «Methoden der organischen Chemie» (Houben Weyl), Band VIII, 274–277), von der Reaktion her einfachste Möglichkeit zum Aufbau des $C_8$-Kohlenstoffgerüstes zu nutzen: nämlich ausgehend von der $C_7$-Kohlenstoff-

kette die Einführung des achten C-Atoms mittels einer Cyanhydrinreaktion.

Dieser Weg wurde vermutlich als für die Herstellung von Pharmazeutika nicht gangbar angesehen, da bekanntlich längerkettige Ketone schlecht reagieren, äusserst instabile Cyanhydrine ergeben und da bei deren Zersetzung entstehende(s) HCN und Cyanide extrem toxisch sind.

Diese Reaktion, gemäss der Erfindung, erfolgt nach der Gleichung

Stufe a:

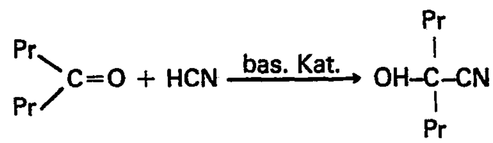

Pr = Propyl

Wird als basischer Katalysator 1 ml gesättigte Ammoniak- bzw. Kaliumcyanidlösung pro 0,5 Mol Di-n-propylketon eingesetzt, so erbringt insbesondere, wenn Kaliumcyanidlösung verwendet wird, die Umsetzung mit Blausäure überraschend hohe Ausbeuten wie 84 Mol-% rohes Di-n-propylketoncyanhydrin.

Für die Reaktion mit wässriger Alkalicyanidlösung gemäss der Gleichung

Stufe a:

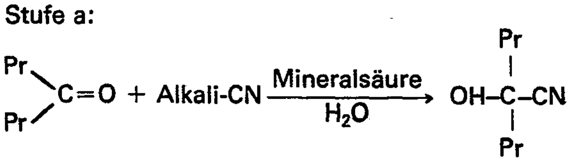


wurden nur geringe Ausbeuten erwartet, wobei die Wasserunlöslichkeit des Ketons, die eine Zweiphasenreaktion zur Folge hat, dazu beiträgt. Überraschenderweise können auch hier gute Ausbeuten erzielt werden. Die Umsetzung von Di-n-Propylketon wird zweckmässig mit Natriumcyanid oder Kaliumcyanid bei Temperaturen von −5 bis +10 °C, vorzugsweise −1 bis +4 °C, in Gegenwart von Mineralsäuren durchgeführt. Als Mineralsäuren eignen sich wässrige Lösungen von $H_2SO_4$, $HNO_3$, HCl, und $H_3PO_4$. Das molare Verhältnis von Cyanid:Schwefelsäure beträgt vorzugsweise 1:0,8 bis 1. Durch die Verwendung einer 68 Gew.-%igen Schwefelsäure kann die Ausbeute auf 71 Mol-% gesteigert werden. Weitere geeignete Massnahmen sind die Herstellung von verdünnten Alkalicyanidlösungen wie 1 Mol Alkalicyanid in 120–200 ml Wasser. So erhält man 86 Mol-% an Rohprodukt, wenn eine derartig verdünnte Natriumcyanidlösung vorgelegt wird. Es können jedoch auch grössere Verdünnungen zum Erfolg führen. Die Reaktanten Di-n-propylketon und Alkalicyanid dienen als Vorlage, zu der die Mineralsäure zugetropft wird. Damit kann eine Ausbeuteverbesserung erzielt werden. Beim Übergang zum Kaliumcyanid wurden 88 Mol-% an destilliertem Produkt gewonnen.

Es ist unerlässlich, das gebildete Di-n-propylketoncyanhydrin zu stabilisieren. Zur Stabilisierung setzt man dem Di-n-propylketoncyanhydrin 0,2 bis 5 Gew.-% konzentrierte starke Mineralsäuren oder starke organische Säuren mit einem pK-Wert von kleiner als 3 zu. Insbesondere seien konzentrierte Mineralsäuren wie konzentrierte $H_2SO_4$ oder $H_3PO_4$ sowie die starken organischen Säuren wie Mono- oder Trichloressigsäure genannt.

Bei zu destillierendem Material ist aufgrund ihrer Flüchtigkeit Chloressigsäure vorzuziehen, die auch in der Dampfphase und Vorlage stabilisierend wirkt. Da erfindungsgemäss eine Destillation von Di-n-propylketoncyanhydrin nicht erforderlich ist, kann mit konzentrierter Mineralsäure, vorzugsweise konzentrierte $H_2SO_4$, stabilisiert werden.

Zur Erzielung guter Ausbeuten ist es zweckmässig, erforderliche Destillationszeiten möglichst kurz zu halten, da trotz Stabilisierung eine langsame Zersetzung des Di-n-propylketoncyanhydrins erfolgen kann. Gemäss dem Verfahren erhält man überraschend gute Di-n-propylketoncyanhydrin-Ausbeuten.

Das Rohcyanhydrin enthält als Verunreinigung lediglich geringe Mengen an Di-n-propylketon, das auch erst nach der Weiterverarbeitung abgetrennt werden kann. Eine Produktreinigung durch Destillation ist daher nicht zwingend erforderlich.

Di-n-propylketoncyanhydrin wird in glatter praktisch einstufiger Reaktion in Gegenwart von konzentrierten Mineralsäuren zu n-Propyl-n-propylidenacetamid umgelagert trotz Einsatzes eines längerkettigen Alkylketoncyanhydrins mit seinen spezifischen sterischen und elektronischen Verhältnissen (Stufe b).

Stufe b:

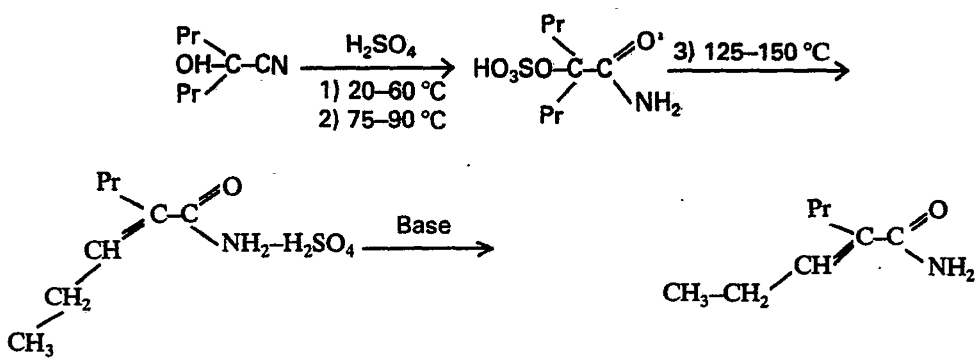

Als konzentrierte Mineralsäure wird in Stufe b vorzugsweise konzentrierte Schwefelsäure eingesetzt, vorzugsweise 96 Gew.-% Schwefelsäure bis 15 Gew.-%iges Oleum. Als optimal im Hinblick auf die Produktausbeute hat sich 98 Gew.-%ige Schwefelsäure erwiesen. Die konzentrierte Mineralsäure wird geeigneterweise in 10–20 Mol-%igem Überschuss, bezogen auf Di-n-propylketoncyanhydrin angewendet.

Die Temperaturführung bei der Umlagerung nach Stufe b wird wie folgt durchgeführt:

1. Vermischung von Di-n-propylketoncyanhydrin mit konzentrierter Mineralsäure, vorzugsweise konzentrierter Schwefelsäure bei 20–60 °C.
2. Anschliessendes Erhitzen auf eine Temperatur von 75–90 °C.
3. Belassen des entstandenen Reaktionsproduktes auf einem Temperaturbereich von 125–140 °C.

Im einzelnen wird dabei so vorgegangen, dass der konzentrierten Mineralsäure unter Einhaltung der obengenannten Temperatur von 20–60 °C Di-n-propylketoncyanhydrin zugemischt wird. Dies hat den Vorteil, dass keine schnelle Dunkelfärbung des Reaktionsgemisches unter Bildung von Polymerisaten auftritt. Vorzugsweise wählt man eine Zumischtemperatur von 50 °C, um eine gute Rührfähigkeit des Kesselinhaltes zu gewährleisten.

Vor dem Zutropfen des Di-n-propylketoncyanhydrins gemäss Stufe b empfiehlt es sich, dem Reaktionsgemisch Inhibitoren zuzugeben, insbesondere Radikalfänger und/oder Metallsalze. Das so beispielsweise mit Hydrochinon und Zinksulfat stabilisierte Gemisch wird nach der Mischung mit konzentrierter Mineralsäure z.B. 1 Stunde bei 80 °C gehalten, wobei geringe Schaumbildung auftritt und die Reaktion zum Schwefelsäureester des 2-Hydroxydipropylessigsäureamides vervollständigt wird.

Danach wird das Reaktionsgemisch z.B. 1 Stunde auf 125–140 °C erwärmt, um Schwefelsäure aus der 2 Position abzuspalten und an die Amidgruppe zum n-Propyl-n-propylidenacetamid anzulagern. Während bei der Anwendung üblicher Reaktionsbedingungen grössere Mengen schwarzer Produkte anfallen, beobachtet man hier nur Braunfärbung.

Nach dem vorliegenden Verfahren wird das Reaktionsprodukt der Stufe b mit wässrigen Basen neutralisiert. Geeignete Basen sind verdünnte Lösungen von Natriumhydroxyd, Ammoniak oder Natriumcarbonat.

Nach Abkühlen auf 80 °C neutralisiert man mit kalter verdünnter Natronlauge oder besser Ammoniaklösung, wobei sich das Produkt auf der Oberfläche in Form gelber Kügelchen abscheidet.

Man rührt kurze Zeit, um eine vollständige Entfernung der Schwefelsäure zu gewährleisten. Nach Abkühlen kann filtriert und mit wenig Wasser salzfrei gewaschen werden. Aufgrund der Schwerlöslichkeit des Produktes entfällt hier das sonst übliche Extrahieren mit organischen Lösungsmitteln. Die in geringer Menge als Nebenprodukt anfallende freie Säure bleibt z.T. als Salz in der Wasserphase gelöst. Das Produkt weist nach dem Trocknen bereits eine Reinheit >98 Mol-% auf und kann durch einmaliges Umkristallisieren aus Lösungsmitteln, vorzugsweise Lösungsmittelgemischen, wie Wasser/Isopropanol oder Petrolbenzin/Toluol oder Sublimieren im Vakuum 99,9 Mol-%ig erhalten werden.

Dieses Ergebnis ist überraschend, da bisher bei der prinzipiell bekannten Umlagerung von Cyanhydrinen (vgl. z.B. DE-C 749 643) die Produkte in wesentlich geringeren Reinheiten anfielen.

Eine Variante des Verfahrens besteht darin, dass man nach der Neutralisiation das Produkt in einem der Wasser nicht mischbaren, für die nachfolgende Hydrierung geeigneten Lösungsmittel wie Di-n-butyläther aufnimmt. Man rührt dann kurz durch, trennt die Phasen und trocknet die organische Phase, die direkt der Hydrierung zugeführt werden kann.

Stufe c:

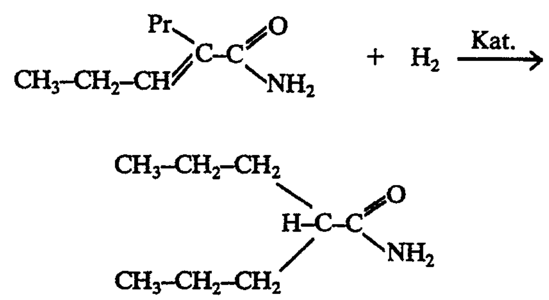


Ist eine Hydrierung zum gesättigten Amid erforderlich, so gelingt diese in nahezu quantitativer Reaktion in an sich bekannter Weise durch Verwendung von 5 Mol-% Pd auf Aktivkohle als Katalysator. Bei 100 °C und 100 bar $H_2$-Druck ist die Hydrierung in Methanol in 30 Minuten beendet. Führt man die Reaktion bei Raumtemperatur durch, wird nur das Z-Isomere hydriert.

Vorteilhaft ist für die Hydrierung die Anwendung eines hochsiedenden Lösungsmittels wie Di-n-butyläther. Diese Massnahme erlaubt eine Hydrierung bei Normaldruck. Beispielsweise erhält man durch Einleiten von Wasserstoffgas in eine ca. 40 Gew.-%-ige Lösung von n-Propyl-n-propylidenacetamid in Di-n-butyläther bei 120 °C in Gegenwart von 3 Mol-% Raney-Nickel als Katalysator nach 4 Stunden quantitativ Di-n-propylacetamid.

Die folgenden Beispiele veranschaulichen die vorliegende Erfindung, ohne sie zu beschränken.

Beispiel 1:

Herstellung von Di-n-propylacetamid

a) Di-n-propylketoncyanhydrin:

Zu 57 g (0,5 Mol) Di-n-propylketon wurde als Katalysator 1 ml gesättigte wässrige KCN-Lösung gegeben und diesem Gemisch bei Raumtemperatur im 250 ml –Dreihalskolben mit Thermometer, Rückflusskühler und Tropftrichter unter Magnetrührung 20 ml (0,55 Mol) wasserfreie Blausäure zugeführt. Um ein Entweichen von Blausäure zu vermeiden, wurde die Apparatur mit einem Überdruckventil verschlossen. Die Innentemperatur

stieg trotz Kühlung mit Eiswasser auf 30 °C. Nach Beendigung der Reaktion wurde mit 2 ml konzentrierter Schwefelsäure stabilisiert, ausgefallenes Salz abfiltriert und nicht umgesetzte Ausgangsmaterialien bei Raumtemperatur am Ölpumpenvakuum entfernt.

Ausbeute: 59,1 g (84 Mol-%, bezogen auf Di-n-propylketon)

b) n-Propyl-n-propylidenacetamid:

In einem 250 ml Vierhalskolben mit KPG-Rührer, Rückflusskühler, Thermometer und Tropftrichter wurden 50 g 98 Gew.-%ige Schwefelsäure und als Polymerisationsinhibitoren 0,4 g Hydrochinon und 0,8 g Zinksulfat vorgelegt. Unter Rühren wurde innerhalb einer Stunde das Di-n-propylketoncyanhydrin so zugetropft, dass ohne Kühlung eine Reaktionstemperatur von 50–55 °C erreicht wurde. Danach wurde langsam auf 80 °C erwärmt und nach dem Abklingen des schwachen Schäumens 1 Stunde bei 130 °C gehalten. Nach Abkühlung wurde mit verdünnter $NH_3$-Lösung bis zur alkalischen Reaktion versetzt, kurze Zeit gerührt, das ausgefallene Produkt abfiltriert, mit wenig Wasser salzfrei gewaschen und getrocknet.

Ausbeute: 46 g (78 Mol-%, bezogen auf Di-n-propylketoncyanhydrin)

Gesamtausbeute, bezogen auf Di-n-propylketon: 65 Mol-%. Produktzusammensetzung (GC): 82,9 Mol-% Z-n-Propyl-n-propylidenacetamid,15,7 Mol-% E-n-Propyl-n-propylidenacetamid, 1,4 Mol-% 2-Propyl-2-pentensäure.

Durch Umkristallisieren aus Petrolbenzin/Toluol 8:2 erhielt man Produkt mit einer Reinheit von 99,9 Mol-%. Weisse nadelige Kristalle, Fp. 86–99 °C, je nach Isomerenverhältnis.

c) Di-n-Propylacetamid:

57,4 g eines rohen n-Propyl-n-propylidenacetamids wurden in 100 ml Methanol gelöst. Nach Zugabe von 0,5 g Katalysator (5% Pd auf Aktivkohle) wurde bei 100 bar $H_2$-Druck und 100 °C im 300 ml Schüttelautoklaven hydriert, bis der $H_2$-Druck konstant blieb (ungefähr 30 Minuten). Nach Abfiltrieren des Katalysators, Abziehen des Lösungsmittels und Trocknen blieben 54,6 g fast weisse Kristalle zurück.

Fp. 124 °C (Lit.: 123–124 °C)

Ausbeute: 94 Mol-%, bezogen auf n-Propyl-n-propylidenacetamid.

Beispiel 2:

Herstellung von Di-n-propylacetamid

a) Di-n-propylketoncyanhydrin

In einem 500 ml-Vierhalskolben (KPG-Rührer, Tropftrichter, Rückflusskühler, Thermometer) wurden 66 g (1 Mol) KCN, gelöst in 120 ml Wasser, und 136,8 g (1,2 Mol) Di-n-propylketon vorgelegt (2 Phasen). Unter kräftigem Rühren wurden innerhalb einer Stunde 135 ml (0,92 Mol) 68 Gew.-%ige Schwefelsäure zugetropft, wobei die Innentemperatur bei −1 bis +1 °C gehalten wurde. Nach 15 Minuten Nachreaktion wurde vom ausgefallenen $KHSO_4$ abfiltriert, die Wasserphase abgetrennt, der Filterkuchen zweimal mit je 100 ml Äther extrahiert, ebenso mit demselben Äther die Wasserphase. Die vereinigten organischen Phasen wurden mit $Na_2SO_4$ getrocknet und nach Abziehen des Äthers über eine 30 cm Vigreux-Kolonne destilliert. Vor der Destillation wurde mit 1 g Chloressigsäure stabilisiert. Der Vorlauf bis 104 °C bei 11 Torr enthielt im wesentlichen Di-n-propylketon neben wenig Di-n-propylketoncyanhydrin. Danach destillierte bei 108 °C und 11 Torr reines Di-n-propylketoncyanhydrin.

Ausbeute: 124,3 g (88 Mol-%, bezogen auf eingesetztes KCN).

b) n-Propyl-n-propylidenacetamid

In der in Beispiel 1 b) beschriebenen Apparatur wurden 60 g 98%ige Schwefelsäure zusammen mit 0,5 g Hydrochinon und 1,8 g Zinksulfat vorgelegt. Unter Rühren liess man ohne Kühlung innerhalb einer Stunde 70 g destilliertes Di-n-propylketoncyanhydrin zutropfen, wobei die Innentemperatur von 20 auf 60 °C anstieg. Das zähflüssige Gemisch wurde langsam auf 80 °C erwärmt und bei dieser Temperatur gehalten, bis kein Schäumen mehr zu beobachten war. (ca. 1 Stunde). Danach wurde 1 Stunde auf 135 °C erhitzt. Nach Neutralisieren mit verdünnter NaOH bei 50 °C wurde weiter gemäss Beispiel 1 b) aufgearbeitet.

Ausbeute: 64,0 g (91 Mol-%, bezogen auf eingesetztes Di-n-propylketoncyanhydrin)

c) Di-n-propylacetamid:

20 g n-Propyl-n-propenylacetamid, 60 ml Methanol und 0,2 g Pd-Katalysator wurden bei 100 bar $H_2$-Druck und Raumtemperatur geschüttelt. Nach Einstellung eines konstanten Drucks (ca. 45 Minuten) wurde eine Probe entnommen und $^1H$-NMR-spektroskopisch und gaschromatographisch untersucht. Ergebnis: Die Z-Form wurde hydriert, die E-Form lag unverändert vor.

Ausbeute (GC) für die Z-Form: 98 Mol-%, bezogen auf n-Propyl-n-propylidenacetamid

Beispiel 3

Herstellung von n-Propyl-n-propylidenacetamid

a) Di-n-propylketoncyanhydrin:

Zur Herstellung des Cyanhydrins wurde eine Versuchsapparatur erstellt, die mit $N_2$ (2 bar) auf Dichtigkeit geprüft wurde.

Im 30 l VA-Kessel wurde 1715 g (35 Mol) NaCN unter Rühren in 7 l Wasser gelöst und anschliessend 4796 g (42 Mol) Di-n-propylketon (BASF; 99%ig) zugegeben. Nach Abkühlen auf 0 °C mit Hilfe von Salzsole (−20 °C) wurden unter kräftigem Rühren (Zweiphasenreaktion!) 4,72 l (31,5 Mol) 60%ige Schwefelsäure innerhalb 105 Minuten zudosiert. Zur Erzielung guter Ausbeuten war eine Reaktionstemperatur zwischen 0 und +2 °C einzuhalten. Dies geschah durch Regelung der Dosiergeschwindigkeit über das Pumpenrad. Nach einer Stunde Nachreaktionszeit wurde der Kesselinhalt zur Entfernung von ausgefallenem $NaHSO_4$ mit $N_2$ in eine VA-Filterpresse gedrückt und das Filtrat in den Kessel zurückgeleitet. Der Filterkuchen wurde mit 5 l Äther extrahiert und der Extrakt ebenfalls in den Kessel gegeben.

Die vereinigten Filtrate wurden im Kessel kurze Zeit durchgerührt und die wässrige Phase in eine Vorlage abgelassen, die zur Neutralisation von gelöster Blausäure 3 l 20%ige Natronlauge ent-

hielt. Ein Schauglas im Auslaufhahn des Kessels ermöglichte die vollständige Abtrennung der wässrigen Phase. Um eine Zersetzung des gebildeten Cyanhydrins beim Erwärmen zu vermeiden, wurden als Stabilisator 50 g konzentrierte Schwefelsäure zugegeben.

Der Kesselinhalt wurde über Warmwasserheizung langsam erwärmt und der Äther bei Normaldruck bis zu einer Kesseltemperatur von 50 °C weitgehend abdestilliert (Vorsicht: Äther blausäurehaltig! Abgasleitung über NaOH-Wäscher führen).

Über eine Ölpumpe wurde dann langsam gesteigertes Vakuum angelegt, wobei der restliche Äther in der Kühlfalle auskondensierte. Anschliessend destillierte überschüssiges Di-n-propylketon über (kp. 20 °C bei 5 Torr). Keton und Äther wurden gesammelt und der Wiederverwendung zugeführt. Die Destillation wurde abgebrochen, als die Kopftemperatur etwa 60 °C erreichte (Kesseltemperatur etwa 75 °C). Der Kesselinhalt wurde entleert und das so gewonnene Di-n-propylketoncyanhydrin dem zweiten Reaktionsschritt zugeführt:

Ausbeute: 4,25 kg (86 Mol-%, bezogen auf eingesetztes NaCN)

b) n-Propyl-n-propylidenacetamid

In einem dampfbeheizten emaillierten 40-l-Kessel wurden 6,8 kg 98%ige Schwefelsäure und als Inhibitoren 100 g Zinksulfat und 50 g Hydrochinon vorgelegt. Unter Rühren liess man bei einer Kesseltemperatur von 50 °C innerhalb einer Stunde 8 kg Di-n-propylketoncyanhydrin zulaufen. Aufgrund der temporär hohen Viskosität des Reaktionsmediums war ein Rührer entsprechender Eignung vorzusehen.

Nach beendeter Substratzugabe wurde das Reaktionsgemisch 1 Stunde bei 80 °C gehalten, wobei geringe Gasentwicklung auftrat (Abgasleitung über NaOH-Wäscher führen!). Danach wurde weiter bis 130 °C erhitzt und der Kesselinhalt 1 Stunde bei dieser Temperatur belassen.

Man liess nunmehr auf etwa 80 °C abkühlen, setzte 20 l kaltes Wasser zu und neutralisierte durch Einleiten von Ammoniak. N-Propyl-n-propylidenacetamid schied sich in Form gelber Kügelchen auf der Oberfläche ab. Nach beendeter Neutralisation wurde der Kesselinhalt unter Rühren abgelassen, das Produkt über eine Filternutsche abgetrennt, mit wenig Wasser salzfrei gewaschen und im Vakuum getrocknet.

Ausbeute: 6,26 kg (78 Mol-%, bezogen auf Di-n-propylketoncyanhydrin).

Beispiel 4

Herstellung von Di-n-propylacetamid

a) n-Propyl-n-propylidenacetamid

In der in Beispiel 1 b) beschriebenen Apparatur wurden 40 g 98 Gew.-%-ige Schwefelsäure, 0,3 g Hydrochinon und 0,6 g Zinksulfat vorgelegt. Unter Rühren liess man 50 g gemäss Beispiel 2 a) hergestelltes destilliertes Di-n-propylketoncyanhydrin zutropfen. Durch Regeln der Dosiergeschwindigkeit wurde ein Anstieg der Reaktionstemperatur auf über 58 °C vermieden. Die weitere Temperaturbehandlung erfolgte analog Beispiel 2 b). Nach Abkühlen auf 80 °C wurden 75 ml Wasser zugegeben und das Reaktionsgemisch durch Einleiten von gasförmigem Ammoniak neutralisiert. Nach Zufügen von 75 ml Di-n-butyläther wurde noch kurz gerührt, bei 60 °C die Phasen getrennt und die organische Phase mit Natriumsulfat getrocknet. Die Lösung wurde ohne Produktisolierung weiter verarbeitet.

b) Di-n-propylacetamid

Von käuflichem, in Wasser suspendiertem Raney-Nickel wurde durch mehrfaches Behandeln mit Methanol, dann mit Di-n-butyläther das Wasser entfernt. In der Apparatur von Stufe a) wurde der Tropftrichter durch ein Gaseinleitungsrohr ersetzt und die in Stufe a) erhaltene Lösung vorgelegt. Nach Zugabe von 0,6 g des Raney-Nickel-Schlammes wurde unter Rühren bei einer Reaktionstemperatur von 120 °C Wasserstoffgas eingeleitet. Die Wasserstoffaufnahme war nach 4 Stunden beendet. Der Katalysator wurde heiss abfiltriert und das nach dem Erkalten auskristallisierte Produkt abgepresst. Insgesamt erhielt man nach dem Aufarbeiten der Mutterlauge 44,8 g Di-n-propylacetamid.

Ausbeute: 88,5 Mol-%, bezogen auf Di-n-propylketoncyanhydrin.

**Patentansprüche**

1. Verfahren zur Herstellung von n-Propyl-n-propylidenacetamid oder Di-n-propylacetamid durch säurekatalysierte Umlagerung von aus Keton hergestelltem Cyanhydrin und gegebenenfalls Hydrierung des ungesättigten Carbonsäureamids, dadurch gekennzeichnet, dass

a) Di-n-propylketon in erster Stufe mit einem Alkalicyamid oder mit Blausäure umgesetzt und

b) das entstehende Di-n-propylketoncyanhydrin in der Weise zu n-Propyl-n-propylidenacetamid umgelagert wird, dass das Cyanhydrin konzentrierter Mineralsäure bei 20–60 °C zugesetzt, anschliessend daran auf eine Temperatur von 75–90 °C erhitzt und schliesslich das entstandene Reaktionsprodukt noch auf 125–140 °C belassen wird, sowie gegebenenfalls nach Abkühlung und Isolierung des Produktes

c) letzteres zu Di-n-propylacetamid hydriert wird.

2. Verfahren nach Anspruch 1, dadurch gekennzeichnet, dass die Umsetzung von Di-n-propylketon mit Natrium- oder Kaliumcyanid bei Temperaturen von −5 bis +10 °C vorzugsweise −1 bis +4 °C in Gegenwart von Mineralsäuren durchgeführt wird.

3. Verfahren nach Anspruch 1 und 2, dadurch gekennzeichnet, dass man dem Di-n-propylketoncyanhydrin 0,2 bis 5 Gew.-% konzentrierte starke Mineralsäuren oder starke organische Säuren mit einem pK-Wert von kleiner als 3 zusetzt.

4. Verfahren nach Anspruch 1 bis 3, dadurch gekennzeichnet, dass in der Stufe b) als konzentrierte Mineralsäure vorzugsweise konzentrierte Schwefelsäuren eingesetzt werden, vorzugsweise 96 Gew.-% Schwefelsäure bis 15 Gew.-%-iges Oleum.

5. Verfahren nach Anspruch 1 bis 4, dadurch gekennzeichnet, dass vor der Umlagerung gemäss Stufe b) Inhibitoren zugegeben werden, insbesondere Radikalfänger und/oder Metallsalze.

6. Verfahren nach Anspruch 1 bis 5, dadurch gekennzeichnet, dass das Reaktionsprodukt der Stufe b) mit wässrigen Basen neutralisiert wird.

**Revendications**

1. Procédé pour préparer le n-propyl-n-propylidène-acétamide ou le di-n-propyl-acétamide par transposition, catalysée par un acide, de la cyanhydrine préparée à partir de la cétone et éventuellement hydrogénation du carboxamide insaturé, procédé caractérisé en ce que:

a) on fait réagir, dans une première étape, la di-n-propyl-cétone avec un cyanure de métal alcalin ou avec l'acide cyanhydrique et

b) on transpose la di-n-propyl-cétone-cyanhydrine formée de manière à la convertir en le n-propyl-n-propylidène-acétamide, cela en ajoutant la cyanhydrine, à 20–60 °C, à un acide minéral concentré, puis en chauffant à 75–90 °C et enfin en maintenant encore à 125–140 °C le produit réactionnel formé, et éventuellement, après refroidissement et isolement du produit:

c) on hydrogène ce dernier pour le convertir en le di-n-propyl-acétamide.

2. Procédé selon la revendication 1, caractérisé en ce que la réaction de la di-n-propyl-cétone avec le cyanure de sodium ou le cyanure de potassium est effectuée à des températures de −5 à +10 °C, de préférence de −1 à +4 °C, en présence d'acides minéraux.

3. Procédé selon l'une des revendications 1 et 2, caractérisé en ce qu'on ajoute à la di-n-propyl-cétone-cyanhydrine de 0,2 à 5% en poids d'acides minéraux forts concentrés ou d'acides organiques forts ayant un pK inférieur à 3.

4. Procédé selon l'une quelconque des revendications 1 à 3, caractérisé en ce qu'on utilise comme acide minéral concentré, dans l'étape b), de préférence un acide sulfurique concentré, plus particulièrement un acide sulfurique dont la concentration va de 96% en poids à celle qui correspond à un oléum à 15% en poids.

5. Procédé selon l'une quelconque des revendications 1 à 4, caractérisé en ce qu'on ajoute des inhibiteurs avant la transposition de l'étape b), ces inhibiteurs étant plus particulièrement des capteurs de radicaux et/ou des sels métalliques.

6. Procédé selon l'une quelconque des revendications 1 à 5, caractérisé en ce qu'on neutralise le produit réactionnel de l'étape b) par des bases aqueuses.

**Claims:**

1. Process for the production of n-propyl-n-propylideneacetamide or di-n-propylacetamide by acid catalyzed conversion of cyanohydrin, which was produced from ketone, and, optionally, hydrogenation of the unsaturated carboxylic acid amide, characterized in that

a) in a first stage, di-n-propyl ketone is reacted with an alkali metal cyanide or hydrocyanic acid, and

b) the resulting di-n-propyl ketone cyanohydrin is converted to n-propyl-n-propylideneacetamide in such a manner that said cyanohydrin is added to a concentrated mineral acid at a temperature of from 20 to 60 °C, subsequently it is heated to a temperature of from 75 to 90 °C, and the resulting reaction product is then left to stand at a temperature of from 125–140 °C, as well as, optionally, after cooling and isolating the product,

c) the latter is hydrogenated to di-n-propylacetamide.

2. Process according to claim 1, characterized in that the reaction of di-n-propyl ketone with sodium cyanide or potassium cyanide is carried out at temperatures of from −5 to +10 °C, preferably from −1 to +4 °C, in the presence of mineral acids.

3. Process according to claim 1 and 2, characterized in that 0.2 to 5% by weight of concentrated strong mineral acids or strong organic acids having a pK value of less than 3 are added to the di-n-propyl ketone cyanohydrin.

4. Process according to claims 1 to 3, characterized in that, in stage b), concentrated sulfuric acid, preferably 96% by weight sulfuric acid to 15% by weight oleum, is preferably used as the concentrated mineral acid.

5. Process according to claims 1 to 4, characterized in that, prior to the conversion according to stage b), inhibitors, especially radical scavengers and/or metal salts, are added.

6. Process according to claims 1 to 5, characterized in that the reaction product of stage b) is neutralized with aqueous bases.